Europäisches Patentamt

**European Patent Office**

Office européen des brevets

(11) Publication number: **0 051 752**
A2

## EUROPEAN PATENT APPLICATION

(21) Application number: **81108154.6**

(22) Date of filing: **09.10.81**

(51) Int. Cl.³: **A 61 K 7/16, A 61 K 7/26**

(30) Priority: **12.11.80 JP 159944/80**

(43) Date of publication of application: **19.05.82**
**Bulletin 82/20**

(84) Designated Contracting States: **DE FR GB IT**

(71) Applicant: **Liau, Weilin, 77-16, Aza-Honcho, Utashinai-Shi Hokkaido (JP)**

(72) Inventor: **Liau, Weilin, 77-16, Aza-Honcho, Utashinai-Shi Hokkaido (JP)**

(74) Representative: **Haft, Uwe Michael, Dipl.-Phys. et al, Patentanwälte Haft, Berngruber, Czybulka Hans-Sachs-Strasse 5, D-8000 München 5 (DE)**

(54) **Dentifrice, its method of manufacture and gargle.**

㊖ The present invention relates to dentifrice and its method of manufacture whereby the dentifrice containing tannic acid efficacious for the prevention of tooth decay, pyorrhea and gingivitis and, at the same time for preventing teeth from being dyed black can be obtained by means of adding to materials for dentifrice what has been treated with reduction by adding copper-chlorophylline-natrium to the said acid and by mixing them together.

EP 0 051 752 A2

ACTORUM AG

## BACKGROUND OF THE INVENTION

The present invention relates to the dentifrice containing tannic acid which is efficacious for the prevention of tooth decay, pyorrhea and gingivitis and to its method of manufacture.

In the field of dental treatment, studies are being carried out of manufacturing oral medicines or dentifrice containing fluorides with the object of preventing or diminishing prevalence of tooth decay. Adding a small amount of fluorine or a small quantity of sodium fluoride into water to drink and dentifrice etc. is also being made as a general preventive measure against tooth decay. Although fluorine is efficacious for strengthening enamel of teeth of infants etc. who are about to cut their milk or permanent teeth, it is impossible to get rid of causes for tooth decay by the help of fluorine only and it is inefficacious to apply fluorine etc. when tooth decay has already been in an advanced stage.

The inventor of this invention, having discovered that tannic acid which is in use as a hemostatic agent for its astringent function has an excellent effect on the prevention and treatment of tooth decay, aimed at making use of this as a dentifrice agent for the prevention of tooth decay. In other words, this inventor has experimentally confirmed that tannic acid (1) is innoxious

- 2 -

unlike fluorine, (2) can be used irrespective of infants and grownups without limitation of the available period unlike fluorine, (3) is harmless to tooth germs even under pH7 and (4) is extremely efficacious for obstruction of advancement of caries of teeth (tooth decay), and has invented a method of manufacture of dentifrice material by means of mixing tannic acid into basic materials for dentifrice in an appropriate way (Patent Gazette SHO-54-38180).

In this connection, a problem arises in a fact that discoloration of teeth takes place when tannic acid is mixed into dentifrice agent. When tannic acid on the market is added as it is to basic materials of dentifrice, tooth germs etc. are dyed black or brown which raises a question when it is used regularly as a preventive agent against tooth decay or as dentifrice.

## SUMMARY OF THE INVENTION

The present invention has solved such a problem as mentioned above and it aims at making an offer of dentifrice containing tannic acid which is efficacious for prevention of tooth decay and, in addition, does not dye teeth black.

The essential points of the invention for achieving the aim mentioned above are to make the basic materials for dentifrice contain tannic acid treated with reduction and to add copper-chlorophylline-natrium (chlorophyll) as a reducing agent.

Other point of the invention lies in the method of manufacture of dentifrice and this is c h a r a c t e r - i z e d by the fact hot water is added to what is composed by adding 0.1 - 0.5% copper-chlorophylline-natrium as a reducing agent to tannic acid equivalent to 1.4 - 2.6% of the total weight of dentifrice which is, after it has cooled down, added to the basic materials of dentifrice followed by mixing them together.

Another point of the present invention is c h a - r a c t e r i z e d by the fact that what is composed by adding copper-chlorophylline-natrium to tannic acid as a reducing agent is contained in gargle.

### BRIEF DESCRIPTION OF THE DRAWING

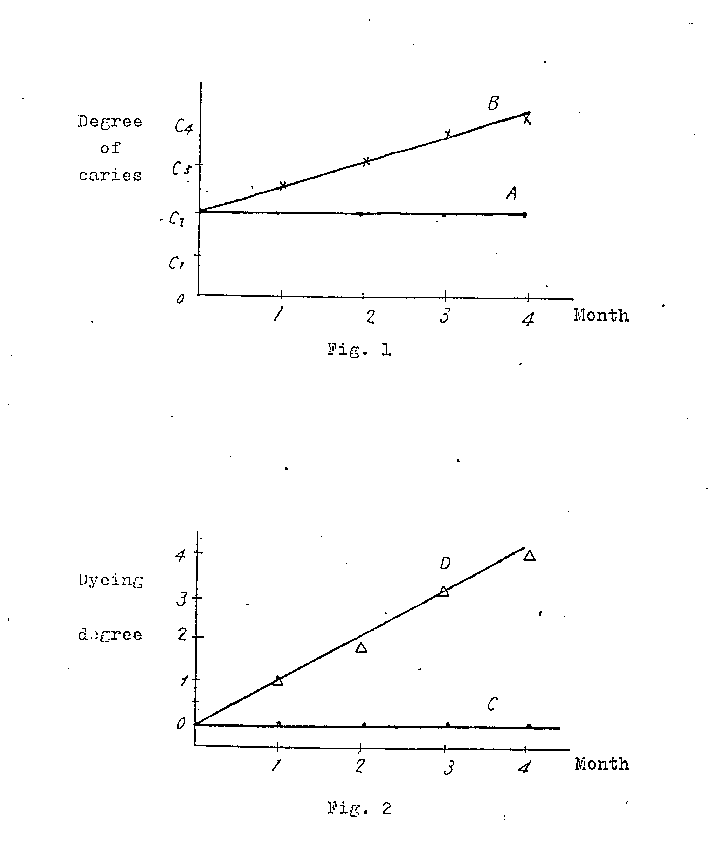

Fig. 1 is a graphic chart showing the degrees of advancement of caries when tannic acid is used calculated by the month and Fig. 2 is a graphic chart showing progress of dyeing of tooth germs by tannic acid.

### DETAILED DESCRIPTION OF THE INVENTION

Copper-chlorphylline-natrium is specially efficacious for halitosis, traumas and stomatitis, functions as a reducing agent when added to tannic acid and restores tannic agent to natural tannic acid (tannic acid contained

- 4 -

in tea leaves and in such tropical plants as betel nuts and leaves of acacia confusa). Accordingly, dentifrice containing tannic acid reduced by copper-chlorophylline-natrium in its basic materials does not dye teeth black and is efficacious for the prevention of tooth decay and pyorrhea when it is used.

The present invention will be illustrated by some practical examples in the following:

Example 1:

(Clinical demonstration)

This inventor infiltrated tannic acid treated with reduction by means of adding 0.1% copper-chlorophylline-natrium to 5% tannic acid into tampons, filled up cavities of decayed teeth of 2 - 3 degrees of caries with them which were sealed with the stoppings, exchanged the tampons for new ones every week, removed the stoppings to uncover the teeth after the tampons were exchanged 16 times and investigated the teeth every month for 4 months. It was discovered as a result of the investigations that the affected teeth remained unchanged at 2 - 3 degrees of caries just the same as the decayed teeth were treated while the teeth of the objects of investigations into which tampons containing tannic acid had not been inserted were found crumbled at their crowns with only the remains of roots of the teeth left behind.

Fig. 1 is a comparative diagram showing the relations between the degrees of caries advancement and the months when tampons of 5% tannic acid with 0.1 copper-chlorophylline-natrium were used (A) and when such tampons were not employed (B). $C_1$ - $C_4$ on the vertical axis are international indication showing the degrees of advancement of tooth decay, that is to say:

$C_1$: A case where decay has advanced up to enamel.

$C_2$: A case where decay has advanced up to cement.

$C_3$: A case where decay has advanced up to dentine.

$C_4$: A case where decay has advanced up to crown with only root left.

The degrees of caries advancement of teeth are bigger as the number of degrees becomes bigger.

Fig. 2 shows a result of discoloration experiments after 4 months when tampons containing tannic acid were inserted to the cavities of decayed teeth of 2 - 5 caries degrees of 10 patients. These tampons were sealed with the stoppings and were exchanged for new tampons every week. "C" in the figure shows the case where 0.1% copper-chlorophylline-natrium was added to 0.5% tannic acid (5 patients) while "D" shows the case where only 5% tannic acid was added without copper-chlorophylline-natrium added (5 patients).

The dyeing degrees by tannic acid of surfaces of teeth are shown on the vertical axis in terms of numerical
values from 0 to 4.

O: No discoloration observed.

1: Dyed yellow.

2: Dyed dark yellow.

3: Dyed brown.

4: Dyed dark brown or black.

As the figures indicate, no discoloration was observed
among the group of partients for whom tannic acid treated
with reduction with copper-chlorophylline-natrium was
added while a remarkable blackening was observed in the
group of patients treated with tannic acid which was not
treated with reduction.

In parallel with the above dyeing experiment, this
inventor conducted daily gargling experiments on voluntary
patients and non-patients with gargle containing tannic
acid with copper-chlorophylline-natrium added and gargling
water containing only tannic acid.  This inventor obtained
quite the same results as those those mentioned above.

As tannic acid treated with reduction proves excellently efficacious clinically as mentioned above, tannic
acid with copper-chlorophylline-natrium added plays
a role similar to that of vaccine when it is mixed into
the base of dentifrice and used for a long time and
it controls the advancement of tooth decay and yet it does not bring about discoloration of teetch. Tannic acid with copper-chlorophylline-natrium added has affinity toward lactic acid and has astringent effects immediately after it has infiltrated into tooth capillaries and is deposited. Basically, pH value of tannic acid at the final formative stage of dentifrice must be less than pH5 so that excessive acid may not hurt teeth. And experiments reveal that tannic acid does not do damage to teeth even when pH value is lower than pH5. The pH of 1% tannic acid is 4.4 (according to the Mohs hardness measurement) and that of 2% tannic acid is pH 3.4. However, it is possible for us to hold the value at pH 5 - 7 by means of adding alkaline detergent in order to prevent meddling of any alien acid when dentifrice is being manufactured. Accordingly, when tannic acid with copper-chlorophylline-natrium added is mixed into the base of dentifrice and is used for a long time, the tannic acid gets astringent and we can get the same effects as those at the time of instillation of 5% tannic acid into a cavity of a decayed tooth conducted at the experiments as mentioned above due to its depositing function.

The table below shows the experimental values of the effectiveness of gargling with 1% tannic acid with copper-chlorophylline-natrium added against pyorrhea and gingivitis.

0051752

TABLE

| No. of days of gargling | No. of people who gargled | Efficacious | Not quite efficacious | Inefficacious |
|---|---|---|---|---|
| 2 | 18 | 2 | 4 | 12 |
| 3 | 18 | 4 | 9 | 5 |
| 4 | 18 | 13 | 3 | 2 |
| 5 | 18 | 16 | 1 | 1 |
| 6 | 18 | 17 | 0 | 1 |

In the table above, the frequency of gargling is about several times a day with 1% tannic acid treated with reduction. "Efficacious" and "Inefficacious" etc. are classified in the following way. As to pyorrhea and gingivitis, dental specialists can tell their respective symptoms and therapeutic values at once with naked eye or by touch. However, when these are to be indicated in terms of numerical values, besides taking measurement of the amount of pus coming out of diverticula of alveoli, there is such an easier way determining symptoms as irrigation of the affected parts with oxydol. When much bubbles are formed at that time, it means that the condition is worsened and when no bubble is formed, it means that the affected part has been cured. In other words, when much amount of bubbles are formed against one drop

- 9 -

of oxydol, it means that the treatment remains "inefficacious", when bubbles are formed in medium degree, it means that the treatment is "not quite efficacious" and, finally, when bubbles are not formed at all, it means that the treatment is "efficacious".

As another example, this inventor achieved good results from experiments of gargling on 2,400 school children. A good result was also observed for the prevention of tooth decay of milk teeth. Although these experiments now mentioned were chiefly conducted on children, the same efficaciousness can also be expected from grownups.

Example 2:

(Example of manufacture of tooth powder)

This inventor succeeded in obtaining the target tooth powder by means of dissolving what is composed by adding 1 g of copper-chlorophylline-natrium (1%) as against 2 g of tannic acid (2%) in 4 cc of hot water, adding, after it has cooled down, 5 g of glycerine to it which was uniformly mixed together, putting it into 0.2 g of menthol dissolved in 1 cc of ether, putting then 0.1 g of soluble saccharine, 10 cc of 2% boric acid, 0.01 g of coloring matter and 1.5 g of aromatic into it, mixing them together uniformaly and finally putting into the above 50 g of precipitating calcium carbonate, 1 g of lauryl sulfate of

0051752

soda and 3 g of medicated soap followed by mixing them together.

Example 3:

(Example of manufacture of toothpaste)

This inventor succeeded in obtaining target toothpaste or solid dentifrice by means of dissolving 2 g of tannic acid (2%) to which 0.1% water solution of copper-chlorophylline-natrium was added in 33 cc of hot water, adding, after it has cooled down, 35 g of glycerine to it, putting it into 0.2 g of menthol dissolved in 1 cc of ether, putting then 0.1 g of soluble saccharine, 10 cc of boric acid (2%), 0.005 g of coloring matter and 1.5 g of aromatic, respectively, into it followed by mixing them together and finally by adding 50 g of precipitating calcium carbonate, 5 g of amylum and 3 g of medicated soap, respectively, to the above which was mixed uniformly.

Example 4:

(Example of manufacture of gargle)

This inventor obtained target gargle by means of dissolving tannic acid (2%) with 0.1% water solution of copper-chlorophylline-natrium added, in 100 cc of water, putting it into 0.2 g of menthol dissolved in 1 cc of ether or 0.2 g of peppermint oil, adding 0.1 g of soluble saccharine and 2 g of boric acid and finally by mixing them together.

Example 5:

(Example of manufacture of solid gargle)

The solid gargle can be obtained by means of adding 2 g of tannic acid with copper-chlorphylline-natrium added as stated in Example 4, 1970 mg of sodium bicarbonate and 0.02 ml of peppermint oil to 2 g (1 wrapper) or (2 pieces) of ................... and by mixing them together.

Two grams (1 wrapper) of this solid gargle can be used at one time by dissolving it in 100 ml of water or lukewarm water.

## C L A I M S

1. The dentifrice c h a r a c t e r i z e d by its containing tannic acid treated with reduction in the materials for the dentifrice.

2. The dentifrice as stated in item 1 of the Claims for which the treatment with reduction is made by means of adding copper-chlorophylline-natrium.

3. The method of manufacture of dentifrice c h a r a - c t e r i z e d by the facts that hot water is added to what is composed by adding copper-chlorophylline-natrium to tannic acid as a reducing agent and that glycerine is added to it after it has cooled down followed by mixing them together.

4. The method of manufacture as stated in item 3 of the Claims c h a r a c t e r i z e d by the facts that the quantity of the tannic acid is 1.4 - 2.6% of the total weight of the dentifrice while that of the copper-chlorphylline-natrium used as a reducing agent is 0.1 - 0.5% of the total weight of the dentifrice.

5. The gargle containing tannic acid with copper-chlorophylline-natrium added as a reducing agent.

Fig. 1

Fig. 2